Europäisches Patentamt

European Patent Office

Office européen des brevets

(11) Numéro de publication: **0 349 450**
**A1**

## (12) DEMANDE DE BREVET EUROPEEN

(21) Numéro de dépôt: 89420232.4

(51) Int. Cl.⁵: **A 61 F 2/36**

(22) Date de dépôt: 27.06.89

(30) Priorité: 28.06.88 FR 8809042

(43) Date de publication de la demande:
03.01.90 Bulletin 90/01

(84) Etats contractants désignés:
BE CH DE ES GB IT LI

(71) Demandeur: **CERAMIQUES TECHNIQUES DESMARQUEST**
**Tour Manhattan Place de l'Iris 6 La Défense 2**
**92400 Courbevoie (FR)**

(72) Inventeur: **Auclair, Michel**
**9, avenue Marcel Hirbec**
**F-78390 Bois d'Arcy (FR)**

**Prats, Christian**
**5, rue de Dreux**
**F-27000 Evreux (FR)**

(74) Mandataire: **Séraphin, Léon et al**
**PECHINEY 28, rue de Bonnel**
**F-69433 Lyon Cedex 3 (FR)**

(54) **Assemblage ameliore d'une tête céramique sur une tige métallique pour prothèse de hanche.**

(57) Assemblage d'une tête céramique (3) sur une tige métallique (1) pour prothèse de hanche permettant une meilleure répartition des contraintes dans la tête céramique (3), ladite tête céramique (3) comprenant un évidement borgne, en général tronconique, dans lequel vient sd'emboîter l'extrémité mâle (2) de la tige métallique (1), caractérisé en ce que cet emboîtement se fait sans jeu, entre la tête céramique (3) et l'extrémité (2) de la tige métallique (1), sur une partie seulement de la hauteur de l'évidement, et que sur la partie restante de l'évidement il n'y a pas contact entre la tête céramique (3) et l'extrémité (2) de la tige métallique (1).

FIG. 2

EP 0 349 450 A1

Description

## ASSEMBLAGE AMELIORE D'UNE TETE CERAMIQUE SUR UNE TIGE METALLIQUE POUR PROTHESE DE HANCHE

### DOMAINE TECHNIQUE

L'invention se rapporte à l'assemblage d'une tête céramique (en général une rotule) avec une tige métallique, pour prothèse de hanche. Cet assemblage, associé à une seconde partie, dite cavité acétabulaire, constitue une prothèse interne d'articulation fémorale.

### ETAT DE LA TECHNIQUE

Il est connu d'utiliser comme prothèse fémorale un ensemble constitué d'une tête céramique et d'une tige métallique fémorale, ladite tige étant d'un côté implantée dans le fémur et de l'autre emboîtée dans un évidement borgne pratiqué dans la tête céramique. Cet assemblage est en général associé à une cuvette acétabulaire naturelle ou artificielle supportant la rotation de la tête céramique pour former une prothèse interne d'articulation de hanche.

La cavité borgne pratiquée dans la tête céramique (partie femelle) est habituellement tronconique à section circulaire et à faible conicité; la partie mâle de la tige métallique fémorale qui vient s'y emboîter a pratiquement le même angle au sommet, de telle sorte que l'assemblage par emboîtement se fait sans jeu entre les deux pièces, la rotation relative de la tête sur la tige étant évitée par différents dispositifs comme l'emmanchement à force, l'utilisation de colles, le clavetage, le brasage ...

Etant donné que l'angle au sommet du cône est faible, il arrive très souvent que lorsque le praticien met en place les deux pièces, leur alignement ne soit pas rigoureusement correct; ainsi au lieu d'avoir une surface de contact entre les parois desdites pièces femelles et mâles, la pièce mâle est, en réalité, en appui par seulement quelques points sur la paroi de l'évidement de la pièce femelle. Pour les mêmes raisons, il arrive également que la pièce mâle ne s'emboîte pas sur toute la portée prévue de l'évidement de la pièce femelle; ainsi par exemple le sommet de la pièce mâle peut porter à faux en un point de la paroi de l'évidement non situé au fond dudit évidement, et en même temps la paroi de contact de la pièce mâle peut porter à faux en un point de la paroi de l'évidement proche de la périphérie de la tête céramique.

Il arrive aussi que à la fois le cône mâle métallique et l'évidement céramique présentent des défauts de géométrie qui font que le contact entre ces deux pièces ne se fait que par quelques points et que leur mise en place n'est pas correcte.

Tous ces problèmes, liés à la mise en place par emboîtement des pièces mâles et femelles, font qu'en définitive les contraintes mécaniques dues aux efforts extérieurs et subies par la tête céramique sont transmises à la pièce mâle et au fémur seulement par l'intermédiaire de quelques points d'appui entre la pièce mâle et l'évidement de la pièce femelle, au lieu de l'être par une surface de contact. Ainsi, les contraintes supportées par la tête céramique sont considérablement accrues en ces points et sont la cause d'une augmentation très importante du risque de rupture de la tête céramique. Ces ruptures se produisent habituellement dans la zone antérieure, par opposition à la zone profonde, de l'évidement de la tête céramique.

### OBJET DE L'INVENTION

L'objet de l'invention est d'obtenir un assemblage par emboîtement entre le cône mâle de la tige métallique fémorale et l'évidement conique de la tête céramique, tel que l'on évite les risques de rupture de ladite tête céramique dus à un mauvais positionnement et/ou à des défauts de géométrie desdites deux pièces en contact.

Un autre objet sera d'assurer une mise en place rigoureuse de ces deux pièces, lors de leur assemblage par le praticien.

Un autre objet est d'avoir un assemblage prothétique fémoral, constitué d'une tête céramique et d'une tige métallique, qui a une meilleure résistance mécanique à la rupture grâce à une répartition optimale des contraintes dans la tête céramique.

### DESCRIPTION DE L'INVENTION

L'invention est un assemblage d'une tête céramique, comprenant un évidement borgne et un tenon mâle s'y emboîtant usiné à une extrémité d'une tige métallique, l'ensemble obtenu associé à une cuvette acétabulaire formant une prothèse interne d'articulation de hanche, ledit assemblage permettant une répartition optimale des contraintes mécaniques dans la tête céramique est caractérisé en ce que l'emboîtement du tenon mâle se fait sans jeu dans une première zone tronconique de l'évidement, appelée zone profonde de contact, située vers le centre de la tête céramique et ayant un faible angle au sommet et que, dans une deuxième zone de l'évidement, dite de non contact, prolongeant la première en direction de la périphérie de la tête céramique et en débouchant à l'extérieur, le tenon mâle et la paroi de ladite deuxième zone de l'évidement ne soient pas en contact.

Selon l'invention, le tenon ou extrémité usinée de la tige métallique, et l'évidement de la tête céramique dans lequel il s'emboîte, ne sont en contact que par l'intermédiaire au plus d'une partie de leurs surfaces latérales totales et le contact est localisé en fond de cavité. Les parties en contact sont donc :
. en ce qui concerne l'évidement de la tête céramique, la partie de sa paroi latérale située dans la zone la plus profonde de l'évidement, à l'exclusion du fond lui-même. Cette partie est tronconique avec un angle au sommet faible
. en ce qui concerne le tenon s'emboîtant dans l'évidement, la partie de sa paroi latérale située le plus près de son extrémité libre.

Il apparaît ainsi que le tronc de cône de la zone profonde de l'évidement, et le tronc de cône de l'extrémité libre du tenon, coopérant entre eux dans l'assemblage, sont usinés avec un angle identique, aux tolérances près, alors que dans la zone de non contact, les surfaces de l'évidement et du tenon, qui sont en regard mais non en contact, sont totalement différentes.

Les cotes des parois en contact sont telles qu'il n'y a pas de jeu entre elles et que le tenon mâle ne vient pas buter sur le fond de l'évidement. Ainsi, le fond de cavité borgne et la surface sommitale transversale du tronc du cône du tenon mâle ne doivent pas être en contact l'un avec l'autre.

Habituellement, la zone profonde de l'évidement de même que la paroi du tenon mâle venant en contact avec elle, ont une symétrie de révolution autour d'un axe se confondant avec un rayon de la tête céramique. Les parois en contact sont de préférence tronconiques, mais nécessairement avec des angles au sommet pratiquement identiques.

L'angle total au sommet de ce tronc de cône commun est faible et se situe en général entre 3 et 10° et de préférence est voisin de 6°.

La hauteur de la surface portante sur laquelle il y a contact entre la paroi latérale de l'évidement et la paroi correspondante du tenon est en général comprise entre 15% et 80%, de préférence entre 25% et 70%, de la hauteur totale de l'évidement. Cette hauteur est en général comptée à partir d'un point situé, sur l'axe de révolution, à une distance telle que l'on préserve un espace libre de garde entre le fond de l'évidement et le sommet du tenon pour éviter qu'ils ne viennent en butée. Il est important que cet espace ait une épaisseur ne dépassant pas 2 mm. En effet, si cet espace de garde est trop important, les contraintes subies par la tête céramique sont déplacées vers des zones à section élargies ce qui présente des risques de rupture et nuit à l'efficacité du dispositif selon l'invention.

La zone profonde de contact de l'évidement est prolongée en direction de la périphérie de la tête, par une deuxième zone où tout contact avec la tige métallique est soigneusement évité. Cette zone de dégagement est obtenue :

. soit de préférence en usinant l'évidement de la tête céramique dans sa zone de non contact pour en augmenter le diamètre moyen, par exemple usinage selon un tronc de cône d'angle au sommet supérieur à celui du tronc de cône de la zone de contact; la surface du tenon en regard peut être alors tronconique avec un angle au sommet plus petit (dans ce cas le tenon peut être avantageusement usiné selon un unique tronc de cône de profil constant sur l'ensemble de la hauteur des deux zones) ou cylindrique

.soit en usinant le tenon, dans sa zone de non contact, pour en diminuer le diamètre moyen, par exemple selon un tronc de cône d'angle au sommet inférieur à celui du tronc de cône de la zone de contact ou selon un cylindre; l'évidement peut alors conserver un profil tronconique constant sur toute sa hauteur.

Ainsi, selon l'invention, on note qu'au moins l'évidement ou le tenon a un profil qui n'est pas constant sur toute leur hauteur; ce qui veut dire que sur le profil en hauteur d'au moins une de ce deux pièces, on note une rupture de la ligne de pente de la surface extérieure qui est en regard avec la surface de l'autre pièce.

Dans la zone de contact, les parois latérales en regard de l'évidement et du tenon sont en contact par la totalité de leur surface.

Le contact se fait selon un tronc de cône commun, non déformable, et est total dès la mise en place des deux pièces, par simple emboîtement, puisque les surfaces de contact existent dès l'usinage; la hauteur de la zone de contact est également constante dès ladite mise en place.

L'épaisseur de l'espace libre de dégagement existant, dans la zone de non contact, entre la paroi de l'évidement et celle du tenon, n'excède pas en général 2 mm et habituellement 1 mm et même parfois moins, elle est constante ou variable sur la hauteur de ladite zone de non contact suivant que les profils de cette deuxième zone de l'évidement et de la zone du tenon lui faisant face sont semblables, par exemple tous les deux cylindriques mais pas de même diamètre, ou différentes, par exemple l'une est cylindrique, l'autre tronconique.

Cette zone de non contact a une hauteur, mesurée sur l'axe de révolution de l'évidement, qui se déduit de la hauteur de contact vue précédemment; elle est ainsi au miniumum égale à 20% de la hauteur totale de l'évidement diminuée de l'espace libre de garde. Elle est donc complètement différente d'une zone de non contact, que l'on trouve dans l'art antérieur, résultant d'un léger chanfrein pratiqué sur l'arête à l'embouchure de l'évidement; en effet, la hauteur de ce chanfrein n'excède pas en général 1 mm et est en général de 0,5mm.

Une fois l'assemblage réalisé par emmanchement, on évite la rotation de la tête céramique sur l'extrémité de la tige métallique par tous moyens connus, tels que colage, emmanchement à force, calvetage, brasage ...

Les figures 1 à 3 permettront de mieux comprendre l'invention.

La figure 1 représente un assemblage tête céramique - tenon métallique selon l'art antérieur. Les figures 2 et 3 représentent deux façons de réaliser l'invention.

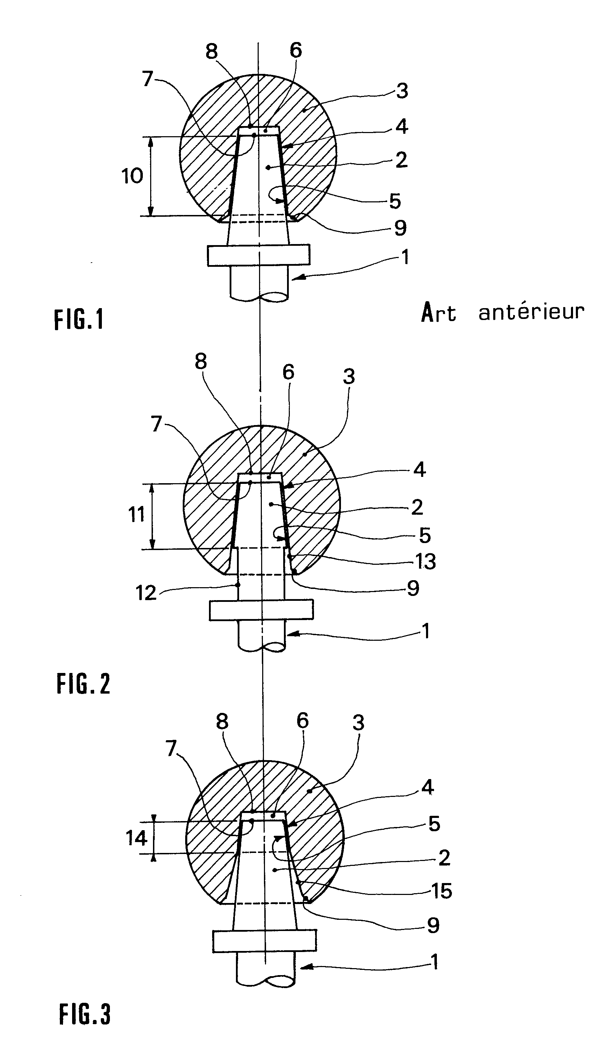

Sur la figure 1 on voit la tige métallique 1 avec son tenon conique usiné 2 destiné à venir s'emboîter entièrement dans l'évidement correspondant de la tête céramique 3. On voit que la paroi 4 de l'évidement et la paroi 5 de l'extrémité de la tige métallique sont en contact sur une hauteur 10 qui est la totalité de la hauteur de l'évidement, diminuée du petit espace de garde 6 séparant le sommet 7 de la tige métallique 1 du fond 8 de l'évidement de la tête céramique 3, et diminuée de la hauteur du chanfrein 9 pratiqué sur l'arête de l'embouchure de l'évidement de la tête 3. Le cône usiné 2 de la tige métallique et l'évidement de la tête céramique 3 étant tronconiques s'emboîtant sans jeu l'un dans l'autre, donnant un assemblage pour prothèse fémorale.

Sur la figure 2, représentant un mode particulier de réalisation de l'assemblage selon l'invention, on voit que l'évidement de la tête céramique est tronconique sur toute sa hauteur. Par contre, le tenon usiné 2 entrant en contact avec la paroi 4 de la zone profonde de l'évidement est tronconique sur une hauteur 11 nettement inférieure à la hauteur totale de l'évidement; ce cône 2 est continué par une partie cylindrique 12 qui fait face sans la toucher à la deuxième zone 13 de non contact de l'évidement. L'épaisseur de l'espace compris entre la paroi de l'évidement et celle de la tige métallique n'est dans ce cas pas constante.

Sur la figure 3 représentant un autre mode particulier de réaisation de l'invention, on voit que l'évidement est toujours tronconique, que la zone profonde de contact 14 est plus faible que dans le cas précédent, et que la deuxième zone de non contact de l'évidement a été obtenue en pratiquant une dépouille tronconique 15 dans la tête céramique, à la suite de la zone profonde, d'angle au sommet légèrement plus grand. Le cône 2 de la tige métallique est tronconique sur toute la hauteur correspondant à l'évidement. Dans ce cas l'épaisseur de l'espace compris entre la paroi de l'évidement obtenue après l'opération de dépouille et la paroi de la tige métallique n'est également pas constante.

L'assemblage selon l'invention est applicable à toutes têtes céramiques frittées à base par exemple d'oxydes tels que les oxydes d'aluminium, de zirconium..., de carbures, de nitrures tels que le nitrures de silicium, de borures... ou leurs mélanges, voire de matériaux composites (par exemple carbone-carbone...). La tête est en général massive, c'est-à-dire ne contient pas d'autre cavité que l'évidemment borgne nécessaire à l'assemblage.
De même, la tige métallique est d'une nature quelconque par exemple titane, inox ou autres alliages, elle peut être également constituée de tous matériaux suffisamment résistants à cette application, par exemple les matériaux composites.
L'invention est appliquée en particulier à la réalisation de prothèses complètes d'articulation de hanche, la cuvette acétabulaire dans laquelle prend place la rotule pouvant être naturelle ou artificielle (en métal, céramique, composite, matière synthétique, etc...) elle est également applicable à toutes liaisons par emboîtement d'une tige métallique dans une cavité borgne d'une tête céramique substantiellement sphéroïdale.

## EXEMPLES

Des essais comparatifs statiques de rupture ont été réalisés sur des têtes correspondant à chacun des cas illustrés dans les figures 1 à 3.
Ils consistent à appliquer une force sur la tête céramique 3, assemblée avec la tige métallique 1-2, selon l'axe de l'assemblage jusqu'à obtenir la ruine de ladite tête céramique; ils ont été effectués selon la procédure décrite dans le projet de norme française PR.S 90443 du 10.2.88 "Pièces fémorales de prothèse de hanche avec tête rapportée: spécifications de la tête et de la partie mâle de l'emboîtement".
Les exemples 1,2,3 correspondent à des essais pratiqués sur des rotules en oxyde d'aluminium frittée de diamètre 28 mm avec un évidement de profondeur 16,5mm; la zone profonde de contact a un profil tronconique avec un fond 8 de diamètre 14mm, et un angle total au sommet de 6°. La tige métallique fémorale est en alliage à base de titane et a un tenon usiné, sa zone de contact a un même angle au sommet que l'évidement, mais le diamètre de son sommet 7 est légèrement supérieur à celui du fond 8 de façon à créer le petit espace de garde 6, entre ledit sommet 7 et le fond 8, dont l'épaisseur est de 1mm.

Les exemples 4 et 5 correspondent à des essais effectués sur des rotules en oxyde de zirconium fritté de diamètre 28mm avec un évidement de profondeur 20mm et un espace de garde 6 d'épaisseur 0,5mm, dans les mêmes conditions que les essais des exemples 1 et 2.

EXEMPLE 1 (fig.1 déjà décrite)
Il illustre un assemblage selon l'art antérieur.
La hauteur de la surface de contact entre le cône 2 de la tige métallique usinée sous forme de tronc de cône et l'évidement correspondant de la tête céramique 3 est de 15mm compte tenu de l'espace de garde 6 et de la hauteur du chanfrein 9 qui est de 0,5mm.
La portée est donc de 100% de la hauteur utile de l'évidement.
Plusieurs essais de rupture ont été effectués; la moyenne de la charge à la rupture obtenue est de 2960 kg.

EXEMPLE 2 (fig. 2 déjà décrite)
La hauteur 11 de la portée est de 10mm, soit 65% de la hauteur totale de l'évidement diminuée de l'épaisseur de l'espace de garde 6.
Comme cela a été dit la zone de non contact a été obtenue par un usinage cylindrique 12, en retrait, de l'extrémité de la tige métallique.
Les mêmes essais ont donné une charge moyenne à la rupture de 5470 kg.

EXEMPLE 3 (fig. 3 déjà décrite)
La hauteur de la portée 14 de contact est de 5mm, soit 33% de hauteur totale de l'évidement diminuée de la hauteur de l'espace de garde 6.
Comme cela a été dit la zone de non contact a été obtenue par usinage tronconique de la deuxième zone de l'évidement, avec un angle légèrement supérieur à celui du tronc de cône de la zone de contact.
Les mêmes essais ont donné une charge moyenne à la rupture de 4600 kg.

EXEMPLE 4 (fig.1, déjà décrite)
Il illustre un assemblage selon l'art antérieur appliqué cette fois à des rotules en oxyde de zirconium fritté. La hauteur de la surface de contact entre le cône 2 de la tihge métallique usinée et l'évidement correspondant de la tête céramique 3 est de 19mm, une fois déduits l'espace de garde 6 de 1mm et la hauteur du chanfrein 9 qui est de 0,5mm.
La portée est de 100% de la hauteur utile de l'évidement.
Les mêmes essais ont conduit à une valeur moyenne de la charge à la rupture de 8750 kg.

EXEMPLE 5 (fig. 2 déjà décrite)

La hauteur 11 de la portée est de 12mm soit 62% de la hauteur totale de l'évidement diminué de l'espace de garde 6.

La zone de non contact a été obtenue par un usinage cylindrique 12 en retrait, du tenon situé à l'extrémité de la tige métallique.

Les mêmes essais ont donné une charge moyenne à la rupture de 12900 kg.

## Revendications

1. Assemblage d'une tête céramique comprenant un évidement borgne et un tenon mâle s'y emboîtant usiné à l'extrémité d'une tige métallique, l'ensemble obtenu associé à une cuvette acétabulaire formant une prothèse interne d'articulation de hanche, et permettant une répartition optimale de contraintes mécaniques dans la tête céramique caractérisé en ce que l'emboîtement du tenon mâle (2) se fait sans jeu dans une première zone tronconique (4,5) de l'évidement, cette première zone dite de contact, étant située vers le centre de la tête céramique et ayant un faible angle au sommet, et que, dans une deuxième zone de l'évidement prolongeant la première en direction de la périphérie de ladite tête céramique (3) et en débouchant à l'extérieur, le tenon mâle (2) et les parois (13,15) de ladite deuxième zone de l'évidement ne sont pas en contact.

2. Assemblage selon la revendication 1, caractérisé en ce que la hauteur de la zone de non contact est obtenue par usinage de la partie en regard correspondante du tenon mâle de la tige métallique.

3. Assemblage sdelon l'une quelconque des revendications 1 et 2, caractérisé en ce que la zone de non contact est obtenue par usinage de l'évidement de la tête céramique dans la zone correspondante.

4. Assemblage selon l'une quelconque des revendications 1 à 3, caractérisé en ce qu'on ménage un espace libre de garde (6) entre le fond (8) de l'évidement borgne et la surface sommitale (7) du tenon (2).

5. Assemblage selon l'une quelconque des revendications 1 ou 4 caractérisé en ce que la hauteur de la zone de contact est comprise entre 15 et 80% et de préférence entre 25 et 70% de la hauteur totale de l'évidement.

6. Assemblage selon la revendication 5, caractérisé en ce que la hauteur de la zone de contact est comptée à partir d'un point situé sur l'axe de révolution du tronc de cône formant la zone de contact, à une distance égale à l'épaisseur de l'espace libre de garde (6).

7. Assemblage selon les revendications 1 à 5, caractérisé en ce que la tête céramique (3) est à base d'oxydes tels que les oxyds d'aluminium, de zirconium..., de carbures, de nitrures, tels que nitrure de silicium, ou de matériaux composites, tels que le carbone-carbone.

8. Assemblage selon l'une quelconque des revendications 1 à 7, caractérisé en ce que l'angle total au sommet de la zone de contact est compris entre 3 et 10° et préférentiellement voisin de 6°.

FIG.1

Art antérieur

FIG.2

FIG.3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 329 249 (SULZER) <br> * En entier * <br> --- | 1,2,4-8 | A 61 F 2/36 |
| X | ENGINEERING IN MEDICINE, vol. 10, no. 4, octobre 1981, pages 175-187, MEP Ltd, Whitstable, Kent, GB; M. KILVINGTON et al.: "In vivo hip joint forces recorded on a strain gauged 'English' prosthesis using an implanted transmitter" <br> * Figure 2 * <br> --- | 1,3,5 | |
| X | DE-A-3 023 354 (SULZER) <br> * Page 3, ligne 26 - page 4, ligne 21; figure 1 * <br> --- | 1,2,4-8 | |
| A | FR-A-2 289 162 (FELDMÜHLE) <br> * Page 4, lignes 12-24; page 9, lignes 5-12; figure 1 * <br> ----- | 1,4,7,8 | |

DOMAINES TECHNIQUES RECHERCHES (Int. Cl.5)

A 61 F

Le présent rapport a été établi pour toutes les revendications

| Lieu de la recherche | Date d'achèvement de la recherche | Examinateur |
|---|---|---|
| LA HAYE | 25-09-1989 | STEENBAKKER J. |

EPO FORM 1503 03.82 (P0402)